# EUROPÄISCHE PATENTANMELDUNG

(11) **EP 2 712 571 A1**
(43) Veröffentlichungstag der Anmeldung: **02.04.2014**
(21) Anmeldenummer: 13186151.0
(22) Anmeldetag: 26.09.2013
(51) Int. Cl.: A61C 3/02, A61C 8/00, A61B 17/14, A61B 17/16, A61C 1/05, A61C 1/08

(54) **Hohlfräse für Dentalzwecke**

(30) Priorität: 26.09.2012 DE 102012018973; 17.10.2012 DE 102012020370
(71) Anmelder: Masur, Ralf, 86825 Bad Wörishofen (DE)
(72) Erfinder: Masur, Ralf, 86825 Bad Wörishofen (DE)
(74) Vertreter: Kruspig, Volkmar

(57) **Zusammenfassung**

Die Erfindung betrifft eine Hohlfräse für Dentalzwecke mit einem Schaft nebst Schaftende und einem Kopfende, wobei am Schaftende ein Einspannbereich und am Kopfende eine Schneide mit einer Vielzahl von Zähnen ausgebildet ist, weiterhin die Schneide in einen Hohlzylinderabschnitt übergeht oder Bestandteil eines solchen Abschnitts ist, wobei außenumfangsseitig der Hohlzylinderabschnitt mindestens eine Fräskante aufweist.

Die Fräskante verläuft drallförmig, wobei der Hohlzylinderabschnitt mindestens eine schlitzförmige Ausnehmung in Axialrichtung besitzt, die dem Drallverlauf der Fräskante folgt.

## Beschreibung

Die Erfindung betrifft eine Hohlfräse für Dentalzwecke mit einem Schaft nebst Schaftende und einem Kopfende, wobei am Schaftende ein Einspannbereich und am Kopfende eine Schneide mit einer Vielzahl von Zähnen ausgebildet ist, weiterhin die Schneide in einen Hohlzylinderabschnitt übergeht oder Bestandteil eines solchen Abschnitts ist, wobei außenumfangsseitig der Hohlzylinderabschnitt mindestens eine Fräskante aufweist, gemäß Oberbegriff des Anspruchs 1.

Aus der WO 2011/026164 A1 ist ein Trepanbohrer für chirurgische, insbesondere zahnchirurgische Zwecke vorbekannt. Dieser Trepanbohrer umfasst einen Bohrerschaft und eine wenigstens teilweise von einem Hohlzylindermantel gebildete Bohrkrone nebst Kühlvorrichtung. Die Kühlvorrichtung besitzt wenigstens einen im Hohlzylindermantel der Bohrkrone verlaufenden Kühlkanal.

Gemäß der vorbekannten Lösung soll die Kühlung von Trepanbohrern verbessert werden, so dass sich die thermische Belastung auch unabhängig von der jeweiligen Bohrtiefe reduziert.

Im Hohlzylindermantel kann wenigstens ein Fenster vorhanden sein, welches zu einer Innenbelüftung und Kühlung des Bohrkopfs führen sowie der einfacheren Entnahme des Knochenkerns dienen soll.

Bei der gattungsbildenden DE 10 2008 029 920 A1 wird ein Trepanbohrer vorgestellt, der einen Schaft mit Einspannbereich sowie einen Kopf aufweist. Der Kopf umfasst einen im Wesentlichen zylindermantelförmigen Kernbohrer, an dessen distalem Ende jeweils eine Schneide mit Zähnen ausgebildet ist. In Umfangsrichtung sind die Zähne alternierend mit unterschiedlicher axialer Zahnhöhe ausgebildet.

Der vorbekannte Bohrer dient z.B. der Gewinnung eines Knochenzylinders aus dem Patientenknochen für die Implantatversorgung. Zur Erzeugung von Hohlzylindern durch Trepane oder Trepanbohrer ist es erforderlich, dass diese ein gleichmäßiges und gutes Schnittverhalten aufweisen und dass der Bohrschritt ohne thermische Schädigung des Knochens eine entsprechende Entnahme eines exakten Knochenzylinders ermöglicht.

Gemäß der Aufgabenstellung nach DE 10 2008 029 920 A1 soll der dortige Trepanbohrer einen einfachen Aufbau besitzen und kostengünstig herstellbar sein, wobei ergänzend ein gutes Schnittverhalten und gute Kühlung besonders an der Außenwand des Trepanbohrers vorhanden sein soll und Weichgewebe zu schonen ist.

Diesbezüglich ist vorgesehen, dass die Verzahnung aus Zähnen aufgebaut ist, welche in Gruppen angeordnet werden. So sind in Umfangsrichtung alternierend unterschiedliche Zähne mit unterschiedlicher axialer Zahnhöhe angeordnet. Die Anordnung der Zähne in Gruppenverzahnung soll ein ruhiges und effizientes Arbeiten ermöglichen. Ergänzend können die Zähne in unterschiedlicher Höhe hinsichtlich ihrer Schneiden und ihrer Schneidgeometrie ausgebildet sein. Bevorzugt sei es, wenn der Spanwinkel in einem Bereich von -20° bis +20° ausgebildet ist.

Bei einer Ausführungsform nach DE 10 2008 029 920 A1 ist der Kopf des Trepanbohrers mit mehreren Ausnehmungen versehen. Die Ausnehmungen sollen der besseren Aufbereitung bei der Reinigung und Sterilisation sowie der Spanaufnahme dienen und die Reibung am Knochenmaterial verhindern. Die Ausnehmungen selbst können z.B. als längliche Schlitze oder als Reihen von runden Ausnehmungen realisiert werden. Bei einer weiteren Ausgestaltung kann die Achse der jeweiligen Ausnehmungen auch um einen Betrag zur Radialrichtung seitlich versetzt werden, so dass sich eine Schneide ausbildet, die einen aggressiven Spanwinkel aufweist.

Es hat sich jedoch gezeigt, dass bei der Ausbildung von schneidenden Kanten an Ausnehmungen im Hohlzylindermantel eine nachteilige Haftung des Knochenmaterials im Inneren des Hohlzylinders die Folge ist.

Aus dem Vorgenannten ist es Aufgabe der Erfindung, eine weiterentwickelte Hohlfräse für Dentalzwecke anzugeben, welche in besonders schonender Weise Säge- bzw. Frässchnitte im Knochen ausführt und wobei dieses auch bei fortschreitender Bohrtiefe möglich ist. Durch die neu zu entwickelnde Hohifräse sollen weitgehend unbeschädigte und thermisch nicht beeinfiusste Knochenblöcke gewinnbar sein. Unter anderem soll der Außenbereich der Knochenbohrung, also das spätere Implantattager thermisch nicht geschädigt werden. Das Ausstoßen von Fragmenten soll leicht und wenig arbeitsintensiv möglich werden.

Die Lösung der Aufgabe der Erfindung erfolgt durch eine Hohlfräse für Dentalzwecke mit einem Schaft nebst Schaftende und einem Kopfende gemäß der Merkmalskombination nach Anspruch 1, wobei die Unteransprüche mindestens zweckmäßige Ausgestaltungen und Weiterbildungen darstellen.

Es wird demnach von einer Hohlfräse für Dentalzwecke mit einem Schaft nebst Schaftende und einem Kopfende ausgegangen, wobei am Schaftende ein Einspannbereich für einen motorischen Antrieb und am Kopfende eine Schneide mit einer Vielzahl von Zähnen ausgebildet ist. Weiterhin geht die Schneide in einen Hohlzylinderabschnitt über oder bildet einen stirnseitigen Bestandteil eines solchen Hohlzylinderabschnitts, wobei außenumfangsseitig der Hohlzylinderabschnitt mindestens eine, bevorzugt mehrere Fräskanten aufweist.

Erfindungsgemäß verläuft die mindestens eine, bevorzugt mehrere Fräskanten drallförmig, wobei der Hohlzylinderabschnitt mindestens eine schlitzförmige Ausnehmung in Axialrichtung besitzt, die dem Drallverlauf der Fräskante folgt.

Durch diese vorgestellte Weiterbildung einer Hohlfräse mit stirnseitigen Zähnen und außenumfangsseitig des Hohlzylinders ausgebildeten Fräskanten ist die Gefahr reduziert, dass sich Späne in den Zähnen sammeln und die Schneidleistung beim Einsatz eines derartigen Trepanwerkzeugs abnimmt. Darüber hinaus werden durch die Schneidenden / Fräskanten und die Aussparungen thermische Schäden am Knochen vermieden.

In einer Ausgestaltung der Erfindung sind außenumfangsseitig am Hohlzylinderabschnitt vier bis acht drallförmig verlaufende Fräskanten ausgebildet, wobei die mindestens eine schlitzförmige Ausnehmung zwischen zwei benachbarten Fräskanten befindlich ist und ebenfalls dem Drallverlauf folgt.

Bei einer bevorzugten Ausführungsform sind im Hohlzylinderabschnitt zwei gegenüberliegende schlitzförmige Ausnehmungen eingebracht. Bevorzugt befinden sich diese beiden schlitzförmigen Ausnehmung zueinander 180° versetzt.

Im Übergangsbereich zwischen dem Hohlzylinderabschnitt und dem Schaft ist mindestens ein kanaiartiger Durchbruch ausgebildet.

Der Drallwinkel der mindestens einen Fräskante liegt im Bereich zwischen 15° und 35°, insbesondere im Wesentlichen bei 20°.

Bei einer weiteren Ausgestaltung der Erfindung kann der Drallwinkel über die Längsausdehnung des Dralls variieren.

Die jeweilige schlitzförmige Ausnehmung im Hohlzylinderabschnitt beginnt in einem Abstand vom Kopfende und endet in einem Abstand vom Ende des Hohlzylinders, verläuft im Übrigen jedoch durchgehend ohne Zwischenstege oder dergleichen brückenartige Verbindungen.

Mindestens eine der drallförmigen Fräskanten weist mit ihrer drehrichtungsabhängigen Frässchneide in Richtung der unmittelbar benachbarten schlitzförmigen Ausnehmung. Knochenmaterial kann hierdurch in die Ausnehmung und damit in das Innere des Hohlzylinders ungehindert eintreten.

Mit der vorgestellten Hohlfräse gelingt eine sichere atraumatische Gewinnung und spätere Platzierung von Knochenmaterial. Mit Hilfe der drallförmigen Fräskanten findet eine Selbstzentrierung beim Einsatz der Hohlfräse statt und es kann bedingt durch die bevorzugt mehreren schlitzförmigen Ausnehmungen Knochenmaterial leicht in das Innere des Hohlzylinderabschnitts eindringen und ebenso leicht entnommen werden.

Die Erfindung soll nachstehend anhand eines Ausführungsbeispiels unter Zuhilfenahme von Figuren näher erläutert werden.

Hierbei zeigen:
- Fig. 1: eine Seitenansicht einer erfindungsgemäßen Hohlfräse für Dentalzwecke mit Schaft und Schaftende sowie Hohlzylinderabschnitt und Kopfende;
- Fig. 2: eine fotorealistische, vergrößerte Darstellung eines erfindungsgemäßen als Hohlfräse ausgebildeten Trepanbohrers und
- Fig. 3: eine Prinzip-Längsschnittdarstellung der erfindungsgemäßen Hohlfräse.

Die Hohlfräse gemäß Ausführungsbeispiel geht von dem Prinzip eines zylinderförmigen Trepanbohrers mit einem zentralen Hohlraum aus, um kreisförmige Schnitte in einem Knochenmaterial auszuführen.

Diesbezüglich weist die Hohlfräse einen Schaft 1 mit einem Schaftende 2 auf, wobei am Schaftende 2 ein Einspannbereich 3 ausgebildet ist.

Am Kopfende 4 befindet sich eine Schneide mit einer Vielzahl von Zähnen 5, wobei die Schneide in einen Hohlzylinderabschnitt 6 übergeht oder Bestandteil eines solchen Hohlzylinderabschnitts 6 ist.

Außenumfangsseitig weist der Hohlzylinderabschnitt 6 mindestens eine Fräskante 7 auf.

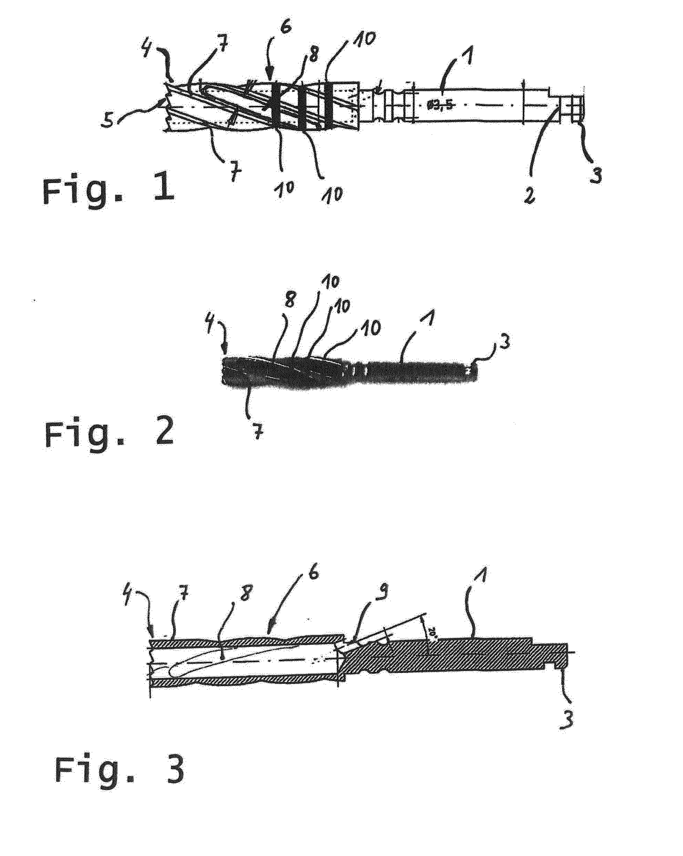

Die mindestens eine Fräskante 7 verläuft drallförmig, wie dies aus den Fig. 1 und 2 ersichtlich ist, wobei der Hohlzylinderabschnitt mindestens eine schlitzförmige Ausnehmung 8, die in Axialrichtung verläuft, besitzt.

Der Verlauf der schlitzförmigen Ausnehmung 8 folgt dem Drallverlauf der Fräskante 7.

Bei einer Ausgestaltung können außenumfangsseitig vier bis acht drallförmig verlaufende Fräskanten 7 ausgebildet sein, wobei die mindestens eine schlitzförmige Ausnehmung 8 zwischen zwei benachbarten Fräskanten 7 befindlich ist und dem Drallverlauf folgt.

Bevorzugt sind im Hohlzylinderabschnitt 6 zwei gegenüberliegende schlitzförmige Ausnehmungen 8 eingebracht (siehe fotorealistische Darstellung nach Fig. 2).

Im Übergangsbereich zwischen Hohlzylinderabschnitt 6 und Schaft 1 ist ein kanatartiger Durchbruch 9 vorhanden, wie dies die Längsschnittdarstellung nach Fig. 3 deutlich macht.

Der Drallwinkel der Fräskanten 7 verläuft in einem Bereich zwischen 15° und 35° und beträgt insbesondere im Wesentlichen 20°.

Der Drallwinkel kann auch über die Längsausdehnung der Hohlfräse variabel ausgeführt sein.

Wie aus den Fig. 1 und 2 ersichtlich, beginnt die jeweilige schlitzförmige Ausnehmung 8 in einem Abstand vom Kopfende 4 und endet in einem Abstand vom Ende des Hohlzylinderabschnitts 6.

Mindestens eine der drallförmigen Fräskanten 7 weist mit ihrer drehrichtungsabhängigen Frässchneide in Richtung der unmittelbar benachbarten schlitzförmigen Ausnehmung 8. Die Ausnehmungen 8 dienen zum einen der besseren Kühlung und der leichteren Entfernung des quasi herausgeschnittenen Knochenzylinders und erleichtern die Reinigung des Trepanbohrers. Beim Einsatz der Hohlfräse bzw. des Bohrers wird quasi ein ganzer Knochenzylinder durch die kopfseitigen Zähne gewonnen, wobei dieser Zylinder in der Regel als ganzes Knochenstück im Inneren des Trepan verbleibt und entnommen werden kann.

Lasermarkierungen 10 bilden eine Bohrtiefenlehre und sind im Hohlzylinderabschnitt 6 vorgesehen.

Mit der Hohlfräse gemäß Ausführungsbeispiel ist eine optimale Kühlung beim Bohr- und Fräsvorgang gegeben, ohne dass Knochenmaterial einer zu hohen thermischen Belastung ausgesetzt wird. Das gewonnene Knochenmaterial lässt sich aufgrund der speziellen Ausbildung der schlitzförmigen Ausnehmungen leicht aus der Fräse und dem Hohlzylinderabschnitt entnehmen und zum Knochenaufbau verwenden.

Durch die flächenmäßig groß ausgeführten schlitzförmigen Ausnehmungen ist es weiterhin möglich, im Bedarfsfall die Hohlfräse klinischen Bedingungen entsprechend zu reinigen und zu sterilisieren, ohne dass es hierfür eines zu hohen Aufwands bedarf.

## Patentansprüche

1. Hohlfräse für Dentalzwecke mit einem Schaft (1) nebst Schaftende (2) und einem Kopfende (4), wobei am Schaftende (2) ein Einspannbereich (3) und am Kopfende (4) eine Schneide mit einer Vielzahl von Zähnen (5) ausgebildet ist, weiterhin die Schneide in einen Hohlzylinderabschnitt (6) übergeht oder Bestandteil eines solchen Hohlzylinderabschnitts (6) ist, wobei außenumfangsseitig der Hohlzylinderabschnitt (6) vier bis acht drallförmig verlaufende Fräskanten (7) aufweist, wobei der Hohlzylinderabschnitt (6) mindestens eine schlitzförmige Ausnehmung (8) in Axialrichtung besitzt, die dem Drallverlauf der Fräskanten (7) folgt und zwischen zwei benachbarten Fräskanten (7) befindlich ist sowie weiterhin im Übergangsbereich zwischen Hohlzylinderabschnitt (6) und Schaft (1) ein kanalartiger Durchbruch (9) ausgebildet ist.

2. Hohlfräse nach Anspruch 1.
**dadurch gekennzeichnet, dass**
der Drallwinkel der jeweiligen Fräskante (7) im Bereich zwischen 15° und 35° liegt, insbesondere 20° beträgt.

3. Hohlfräse nach einem der vorangegangenen Ansprüche,
**dadurch gekennzeichnet, dass**
der Drallwinkel über die Längsausdehnung des Dralls variiert.

4. Hohlfräse nach einem der vorangegangenen Ansprüche,
**dadurch gekennzeichnet, dass**
die jeweilige schlitzförmige Ausnehmung (8) in einem Abstand vom Kopfende (4) beginnt und in einem Abstand vom Ende des Hohlzylinderabschnitts (6) endet.

5. Hohlfräse nach einem der vorangegangenen Ansprüche,
**dadurch gekennzeichnet, dass**
mindestens eine der drallförmigen Fräskanten (7) mit ihrer drehrichtungsabhängigen Frässchneide in Richtung der unmittelbar benachbarten schlitzförmigen Ausnehmung (8) weist.
